(19) Europäisches Patentamt
European Patent Office
Office européen des brevets

(11) Publication number: 0 213 704 A2

(12) # EUROPEAN PATENT APPLICATION

(21) Application number: 86305378.1

(51) Int. Cl.[4]: A61K 31/665 , C07F 9/38

(22) Date of filing: 14.07.86

(30) Priority: 03.09.85 GB 8521832

(43) Date of publication of application: 11.03.87 Bulletin 87/11

(84) Designated Contracting States: AT BE CH DE FR IT LI LU NL SE

(71) Applicant: COMPANIA ESPANOLA DE LA PENICILINA Y ANTIBIOTICOS, S.A.
Paseo de la Castellana 20
Madrid(ES)

(72) Inventor: Torregrosa Higom, Arturo
General Pardinas 69
Madrid(ES)
Inventor: Fernandez Ibanez, Matilde
Almansa 5
Madrid(ES)
Inventor: Garcia Sanchez, Milian
Rios Rosas 6
Madrid(ES)
Inventor: Prieto Prieto, Agustin
Somosierra 10 Las Rozas
Madrid(ES)

(74) Representative: Skailes, Humphrey John et al
Frank B. Dehn & Co. Imperial House 15-19
Kingsway
London WC2B 6UZ(GB)

(54) **Mixtures of mono- and disodium salts of fosfomycin.**

(57) Mixtures of the mono-and di-sodium salts of fosfomycin and their use by parenteral and oral administration are described.

EP 0 213 704 A2

# MIXTURES OF MONO-AND DISODIUM SALTS OF FOSFOMYCIN

This invention relates the parenteral and oral use of mixtures of monosodium and disodium salts of the antibiotic FOSFOMYCIN, chemically designated as (-) (cis-1,2-epoxy-propyl)phosphonic acid. The mixtures subject of this invention are those containing 60-65% of the monosodium salt, of formula

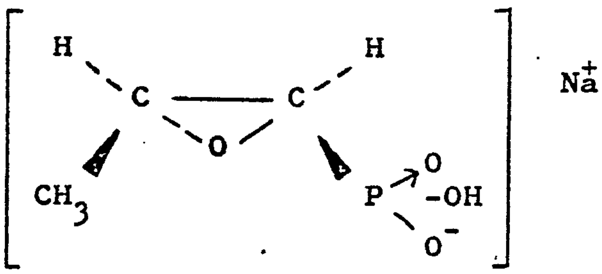

and 40-35% of disodium salt, of formula

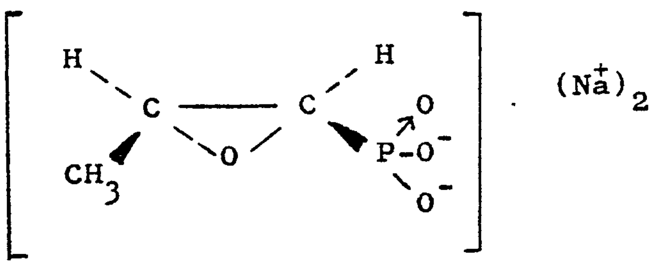

Fosfomycin is an antibiotic discovered in Spain in 1966 which is described in the Merck Index, 10th edition, No. 4137, isolated originally from fermentation broths of a strain of Streptomyces fradiae obtained from a soil sample collected in Jávea, Alicante, Spain.

The mono-salts or di-salts of fosfomycin are active against gram-positive and gram-negative bacteria, and therapeutically useful in the treatment of infections caused thereby. Its antibiotic spectrum comprises the following species:

Aerobes

| | |
|---|---|
| Staphylococcus aureus | Klebsiella spp. |
| Streptococcus pyogenes | Enterobacter spp. |
| Streptococcus pneumoniae | Serratia marcescens |
| Streptococcus faecalis | Pseudomonas aeruginosa |
| Neisseria meningitidis | Salmonella spp. |
| Neisseria gonorrhoeae | Shigella spp. |
| Haemophilus influenzae | Campylobacter fetus jejuni |
| Legionella pneumophila | Yersinia enterocolitica |
| Escherichia coli | Acinetobacter calcoaceticus |
| Indole(-) Proteus spp. | Vibrio cholerae |
| Indole(+) Proteus spp. | Aeromonas spp. |

Anaerobes

| | |
|---|---|
| Peptococcus spp. | Fusobacterium spp. |
| Peptostreptococcus spp. | Clostridium spp. |

The salts of alkaline metals and alkaline-earth metals, such as mono-and di-salts of sodium, potassium, calcium, magnesium, etc., have been known for a long time. Also known are salts formed with amines, such as α-phenethylamine, quinine, lysine, procaine, etc., which can be mono-or di-salts.

The salts of fosfomycin currently used in antibiotherapy are calcium salt for oral administration and disodium salt for parenteral administration - (intramuscular and intravenous).

The mixture of mono and disodium salts (60-65/40-35) displays advantages over those used at present. In parenteral administration, disodium fosfomycin gives very alkaline aqueous solutions, thus requiring the use of acids to achieve a physiological pH, and also increases the tonicity of the solution, and because of this results in painful intramuscular administration, with the additional limitation due to the high sodium content.

The mixture of mono-and disodium salts reduces or avoids the side effects of the disodium salt, as the pH of the solution of the mixture is closer to the physiological pH and therefore the use of neutralizing acids is not required. Moreover the mono and disalt mixture reduces the sodium problem.

In oral administration, the plasma concentrations obtained in a crossed test with the calcium salt and the mixture of mono and disodium salts, at a single oral dose of 1 g, show a significantly higher bioavailability than the calcium and disodium salts.

The following example is illustrative of the process subject of the invention and should not be considered to be limitative.

**Example** : Preparation of the mixture of monosodium salt

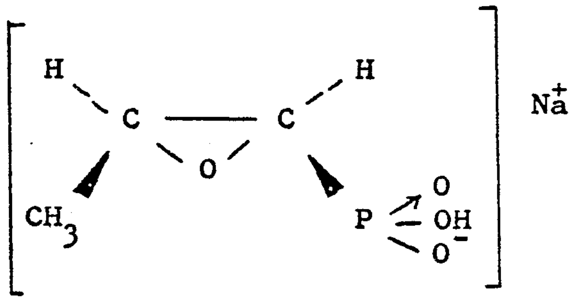

and disodium salt

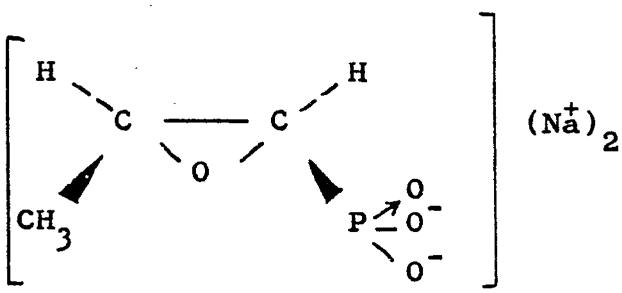

of (-)(cis-1,2-epoxypropyl)phosphonic acid:

Prepare a solution of 20 g of mono (+) -α - phenethylamine salt of fosfomycin in methanol. Add 5.7 g of sodium methoxide and mix for 15 minutes. The mixture of mono-and disodium salts of fosfomycin is precipitated with acetone. About 10 g of the mixture of fosfomycin salts were obtained, being characterized by the parameters detailed below:

N.M.R. (in deuterium oxide)

ppm (δ)

1.48 (d,d; J = 5.7 and 0.7 Hz; 3H, $CH_3$)

2.85 (d,d; J = 5.4 and 21.3 Hz; 1H, CH)

3.30 (m ; J = 5.4 1H, CH)

I.R. Spectrum (in KBr pressed discs)

The IR spectrum displays characteristics bands at 1416, 1380, 1339, 1268, 1200, 1040, 1000, 950, 920, 860, 735 $cm^{-1}$.

pH (5% water solution) : 6.50

Sodium content (%) : 18.2%

Microbiological assay: Against Proteus mirabilis ATCC 21100; antibiotic activity equivalent to 800 µg/mg of free fosfomycin acid.

PHARMACEUTICAL FORMS

Oral administration

CAPSULES (500 mg)

| | |
|---|---|
| Fosfomycin (mono and disodium salts mixture)... | 500 mg |
| Magnesium stearate ........................... | 4 mg |
| Lactose c.s.p. ................................ | 620 mg |

SACHETS (500 mg)

| | |
|---|---|
| Fosfomycin (mono and disodium salts mixture)... | 500 mg |
| Fruit flavour ................................. | 150 mg |
| Sucrose c.s.p. ................................ | 3,500 mg |

SACHETS (1 g)

| | |
|---|---|
| Fosfomycin (mono and disodium salts mixture)... | 1,000 mg |
| Fruit flavour ................................. | 150 mg |
| Sucrose c.s.p. ............................... | 4,200 mg |

Parenteral administration (intramuscular or intravenous)

VIAL 1 g

Fosfomycin (mono and disodium salts mixture).. 1,000 mg

VIAL 4 g

Fosfomycin (mono and disodium salts mixture) ..4,000 mg

For intramuscular administration use ampoules containing local anesthesics, example:

Lidocaine HCl .................. 25 mg

Water for injection c.s.p. ..... 4 ml

## Claims

1. The use of mixtures of fosfomycin monosodium and disodium salts for parenteral and oral administration.

2. Pharmaceutical compositions for oral and parenteral administration, containing as an active ingredient mixtures of monosodium and disodium salts defined in claim 1, together with excipients and/or pharmaceutically acceptable carriers.

3. A process for preparing the mono-and disodium salts of fosfomycin and mixtures thereof, characterized in that the fosfomycin phenethylamine salt is reacted with sodium methoxide in alcoholic medium, and the salts are precipitated by addition of a solvent.

4. A process according to claim 3, wherein the alcohol is slected from a straight-or branched chain-monohydroxyl alcohol with a number of carbon atoms comprised between 1 and 5 of said alcohols, preferably methanol.

5. A process according to claim 3, wherein the precipitating solvent is one of the following: ethyl acetate, acetone, isopropanol, ethanol, petroleum ether, ethyl ether, or a mixture of thereof, preferably acetone or isopropanol.

6. A method for treating human or animal infections consisting of administering thereto one of the pharmaceutical compositions defined in claim 2.